# DEMANDE DE BREVET EUROPEEN

(11) **EP 0 591 097 A1**
(43) Date de publication de la demande: **06.04.1994**
(21) Numéro de dépôt: 93810567.3
(22) Date de dépôt: 10.08.1993
(51) Int. Cl.: A61H 39/00, A61N 5/06

(54) **Appareil de chromo-réflexologie**

(30) Priorité: 28.08.1992 FR 9210508
(71) Demandeur: ELIOPTIC LIMITED, Dublin 2 (IE)
(72) Inventeur: Echeverria, Alexandra, Guayaquil (EC)
(74) Mandataire: Moinas, Michel

(57) **Abrégé**

L'appareil de chromo-réflexologie comprend une source (1) de lumière polychromatique, des moyens (6) pour disperser cette lumière et en réaliser un spectre (10), au moins un guide d'onde (11) reprenant à partir de la lumière dispersée un faisceau monochromatique, l'extrémité de sortie (13) du guide d'onde conduisant la lumière monochromatique à proximité du point de traitement, et des moyens pour balayer le spectre de façon continu avec l'extrémité d'entrée (12) du guide d'onde. On peut sélectionner ainsi et à volonté la longueur d'onde de la lumière reprise dans le guide (11) et conduite vers le point de traitement, qui correspond à une zone réflexe du corps.

## Description

La présente invention se rapporte à un appareil de chromo-réflexologie utilisable aussi bien en médecine humaine, y compris dans les domaines paramédicaux et de l'esthétique, qu'en médecine vétérinaire.

Comme son nom l'indique, la chromo-réflexologie est une technique médicale consistant à traiter le corps humain ou animal en dirigeant une lumière colorée sur les zones réflexes du corps, aussi ponctuellement que possible, ceci afin de provoquer une réaction physiologique chez le sujet. Cette réaction va à son tour induire un soulagement ou la disparition de certaines douleurs, l'amélioration de l'état général du patient et le rééquilibrage de sa situation énergétique. L'emploi même du terme "énergétique" montre bien que la chromo-réflexologie, et que d'une façon plus générale la chromo-thérapie, a sans doute des liens de parenté avec des techniques telles que l'acupuncture. En effet, parmi les zones réflexes qui seront la cible du traitement de chromo-réflexologie figurent les points d'acupuncture, les autres zones réflexes du corps étant notamment situées sur l'iris de l'oeil (iridologie), le pavillon de l'oreille (auriculothérapie), la paume des mains, la plante des pieds, etc...

Ces techniques médicales, dont certaines telles que l'acupuncture sont d'origine non-européenne et remontent à des temps reculés, sont des techniques qualifiées maintenant de "médecine douce", de "médecine naturelle" ou encore de "médecine alternative", et sont utilisées avec succès par tous les praticiens soucieux de traiter le patient, et non seulement la maladie.

Afin d'obtenir un effet en chromo-réflexologie, il convient bien sûr de choisir la zone réflexe servant de cible en fonction des effets recherchés, la zone cible n'ayant généralement pas de lien de proximité immédiate avec la zone douloureuse ou le déséquilibre à traiter. On sait parexemple qu'en acupuncture le point du corps où l'on pique une aiguille n'a généralement pas de rapport évident pour le profane avec la partie corporelle à soigner, l'important étant le rapport qui peut exister à travers un méridien ou un lien énergétique. De la même façon, en iridologie, on traite de façon très précise tel ou tel point sur l'iris de l'oeil, fonction la partie corporelle que l'on veut soigner. Il existe d'ailleurs une cartographie très précise de l'iris quant à ses relations avec les autres parties du corps (parties rectale, génitale, laryngo-thyroïdienne, rhino- ophtalmologie, cardiaque, etc..) Tout ceci est parfaitement connu et codifié.

L'autre problème qui se pose en chromo-réflexologie est, une fois déterminée la zone cible convenant au traitement, de déterminer la longueur d'onde de la lumière susceptible d'agir, étant entendu que la zone réflexe ne sera sensible qu'à une lumière bien définie, fonction généralement et à la fois du patient et de son état général. Il ne sert donc à rien d'illuminer la zone cible par une lumière blanche ou polychromatique, le réflexe n'étant obtenu que pour une longueur d'onde bien déterminée appartenant au spectre de la lumière blanche.

Des appareils destinés à la chromothérapie, ont été mis au point et sont actuellement utilisés. Ils génèrent une lumière blanche, qui est rendue aussi monochromatique que possible à l'aide d'une série de filtres montés sur un plateau tournant. En faisant tourner le plateau, le praticien place un filtre sur le trajet de la lumière blanche, puis un autre, et peut ainsi tour- à-tour sélectionner différentes couleurs.

Il s'agit pas d'appareils pour la chromo-réflexologie, mais pour la chromothérapie, c'est-à-dire pour une technique qui n'utilise pas la notion de zone réflexe et se limite à exploiter une topographie se référant aux symptômes décrits par le patient. Le praticien, après avoir interrogé son patient, applique sur la zone déficiente une couleur monochromatique, mais en suivant des règles immuables ne tenant pas compte de la spécificité individuelle de ce patient, et sans réflexologie de contrôle.

Au contraire, la chromo-réflexologie s'attache à l'étude de la modification de zones, telles que le pouls périphérique radial, l'iris signal, le réflexe pupillaire controlatéral, etc..., en partant de l'excitation d'une zone réflexe périphérique par un faisceau lumineux coloré.

Quoi qu'il en soit, la couleur générée pas ces appareils n'est jamais "pure", c'est-à-dire suffisamment monochromatique, et ces appareils de chromothérapie sont lourds et encombrants.

Comme autre exemple d'appareil utilisant un filtre, on citera à titre anecdotique, celui qui est décrit dans le brevet US-4 505 545, et est destiné en réalité au traitement de certaines déficiences cutanées, musculaires et nerveuses. Outre qu'il s'agit d'autre technique médicale radicalement différente puisqu'on ne vise pas ici les zones réflexes du corps, on observera que la lumière colorée résultant à partir du blanc de l'absorption des couleurs complémentaires par un filtre, est appliquée sur une grande surface.

On citera également, toujours à titre anecdotique, l'appareil décrit dans le brevet FR-2 521 851, qui est en fait n'est pas un appareil à usage de soins. Cet appareil est destiné à faire des macro-photographies de l'iris de l'oeil. L'iris est illuminé et les photographies sont prises, éventuellement à l'aide d'un flash, en utilisant un guide de lumière équipé d'un prisme à réflexion totale. Il ne s'agit nullement d'employer une lumière aussi monochromatique que possible sur un point précis de l'iris de l'oeil.

On citera enfin l'appareil décrit dans l'exposé WO 83/01311 qui comprend un prisme destiné à étaler la lumière, laquelle est reprise à l'aide d'une fibre optique dirigée sur un point particulier du corps. Le but est en l'occurrence d'activer à l'aide de la lumière un produit actif sensible présent dans une tumeur.

L'invention reprend certains des éléments décrits dans l'exposé précédent, mais dans un but tout différent, puisqu'il s'agit d'envoyer sur une zone réflexe du corps une lumière aussi ponctuelle et monochromatique que possible afin d'y déclencher une réaction induisant des effets métaboliques et énergétiques, sans aucune intervention d'un quelconque produit exogène photosensible.

Selon l'invention, l'appareil de chromo-réflexologie comprend une source de lumière polychromatique, par exemple de la lumière blanche, des moyens pour disperser cette lumière et en réaliser un spectre, au moins un guide d'onde reprenant à partir de la lumière dispersée un faisceau monochromatique, l'extrémité de sortie du guide d'onde conduisant la lumière monochromatique à proximité du point de traitement et des moyens pour balayer le spectre de façon continue avec l'extrémité d'entrée du guide d'onde. De la sorte, on peut sélectionner à volonté la longueur d'onde de la lumière reprise dans le guide d'onde et conduire cette lumière vers le point de traitement, qui correspond à une zone réflexe du corps. Typiquement, l'extrémité de sortie de la fibre optique est placée entre 0,5 et 1,5 cm de la zone réflexe à traiter.

Par l'expression "lumière monochromatique", on entend signifier que la lumière est "aussi monochromatique que possible". Théoriquement, seul un faisceau infiniment fin repris du spectre, c'est-à-dire prélevé au sein de la lumière dispersée, peut correspondre à une lumière strictement monochromatique. Pour approcher au maximum la monochromaticité désirée, on reprendra la lumière dispersée par un guide d'onde aussi ponctuels que possible, par exemple en utilisant une fibre optique, avantageusement une fibre optique ayant un diamètre compris entre 1 et 3,5 mm.

Il faut de plus bien comprendre que la lumière monochromatique dirigée sur la zone réflexe est une lumière "froide", c'est-à-dire une lumière de faible puissance ou énergie, n'induisant pas de phénomènes calorifiques.

Il faut bien comprendre aussi qu'il ne s'agit pas d'une lumière cohérente, telle que celle qui serait émise par un laser. Au contraire, le faisceau de lumière coloré qui est envoyé sur la zone réflexe est un faisceau de lumière non cohérente.

Comme moyen de dispersion, on peut utiliser un prisme ou un réseau, étant entendu que le réseau est de loin préférable. Autorisant une meilleure séparation des diverses longueurs d'ondes sur une plus courte distance, il permet des montages beaucoup plus compacts et donc plus faciles à manipuler.

Pour balayer le spectre et en extraire un faisceau de lumière monochromatique, on pourra utiliser tout simplement un balayage par des moyens mécaniques, qui déplacent l'extrémité d'entrée d'onde à l'intérieur du spectre.

De préférence, l'appareil comprend également un disque tournant percé de trous, placé sur le chemin lumineux, afin d'interrompre périodiquement celui-ci et de créer à la sortie un faisceau pulsé. On a constaté en effet que les effets obtenus sont souvent renforcés lorsque le traitement s'effectue à l'aide d'un faisceau pulsé, plutôt que d'un faisceau continu.

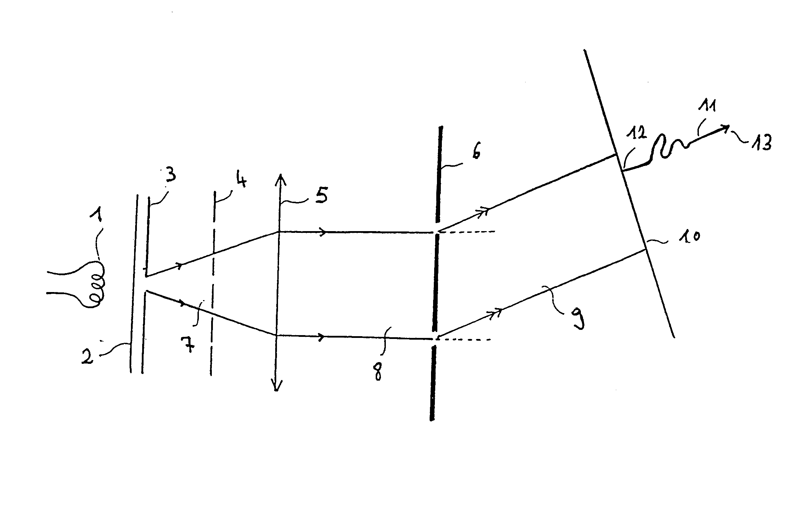

L'invention sera mieux comprise en référence au dessin annexé, figure unique, donné à titre d'exemple non limitatif.

Comme on le voit le schéma de principe donné en figure, l'appareil comprend une source lumineuse 1 sous forme d'un filament, par exemple d'une lampe à incandescence telle qu'une lampe halogène.

En avant de cette lampe est placé un filtre anticalorique 2, de façon à éviter la transmission de chaleur à travers l'appareil. Comme il a été indiqué plus haut, il faut éviter autant que possible les effets calorifiques. En arrière du filtre anticalorique se trouve un diaphragme 3 percé d'une ouverture très fine dans l'axe optique. C'est ce trou qui va agir comme source lumineuse ponctuelle. On rencontre ensuite un disque tournant 4 percé de trous, de façon à pouvoir pulser le faisceau lumineux en l'interrompant périodiquement. Le faisceau passe ensuite à travers une lentille 5, puis tombe sur un réseau 6, qui étale la lumière polychromatique et en crée un spectre coloré 10 qui est repris ponctuellement par une fibre optique 11 via son extrémité d'entrée 12, l'extrémité de sortie 13 de cette fibre 11 étant amenée à proximité de la zone réflexe à traiter.

Le système optique ainsi décrit permet de transformer un faisceau divergent à partir de l'ouverture ménagée dans le diaphragme 3 en un faisceau de lumière parallèle 8, qui va être étalé sous forme d'un spectre 10 par le réseau 6.

A titre d'exemple, la lampe peut être une lampe halogène de 250 W fonctionnant sous une tension de 24 V et le réseau 6 peut comprendre entre 100 et 700 lignes par mm, par exemple 600. Il est avantageusement constitué de fentes parallèles et équidistantes, situées dans un même plan, l'observation de la lumière se faisant à l'infini. Le foyer objet de la lentille 5 est centré sur l'ouverture de diaphragme 3. On peut bien entendu faire varier la vitesse du disque troué tournant 4, des vitesses différentes de rotation permettant la tonification ou la dispersion énergétique, comme cela est bien connu en acupuncture.

Comme indiqué plus haut, cet appareil est utilisé pour diriger une lumière monochromatique au niveau d'une zone réflexe du corps comprenant aussi bien les points d'acupuncture que les zones réflexes proprement dites, comme l'iris de l'oeil, le pavillon de l'oreille, la paume des mains, la plante des pieds, etc...

L'utilisation de l'appareil selon l'invention est en relation avec l'expérience du praticien, qui dirige l'extrémité de sortie 13 de la fibre optique vers la zone réflexe choisie en fonction de ses connaissances et de son diagnostic. Le praticien fait varier alors la longueur d'onde de la lumière monochromatique passant dans la fibre optique en déplaçant l'extrémité d'entrée 12 de celle-ci à l'intérieur du spectre lumineux 10, déplacement qui est effectué par exemple par des moyens mécaniques, avantageusement motorisés. Pendant cette opération, le praticien suit attentivement la réaction physiologique de son patient, en suivant par exemple le pouls. Lorsque la longueur d'onde de la lumière choisie est de celle qui provoque un effet, le pouls du patient s'accélère ou se ralentit brusquement pendant une durée de l'ordre de 30 secondes à 2 minutes, durée pendant laquelle le praticien maintient la lumière dirigée sur la zone réflexe. S'il y a lieu, on peut aussi procéder en suivant la modification du réflexe pupillaire.

L'appareil de chromo-réflexologie selon l'invention peut comprendre, notamment s'il s'agit de travailler sur l'iris de l'oeil, un dispositif optique complémentaire de type loupe ou zoom (objectif à focale variable) permettant de travailler loin de la cornée, par exemple un condenseur réglable donnant une image d'un diamètre compris entre 1 et 1,5 mm à une distance de 15 mm environ. Ainsi équipé, l'appareil rendra plus facile les interventions, par exemple par des praticiens non médecins.

En variante non représentée, l'appareil peut être muni d'une deuxième fibre optique reprenant la lumière dispersée au même point (point d'insertion) que la première fibre optique. L'appareil permettra alors l'éclairage simultané de deux zones différentes par une lumière de même longueur d'onde. Les deux zones pourront toutes deux être des zones réflexes, ou seule l'une d'entre elle le sera. Cette deuxième fibre permettra par exemple d'éclairer l'iris controlatéral, à la recherche d'un signe pupillaire. Son extrémité de sortie distale pourra se terminer par une pièce à main, munie avantageusement d'une loupe de 35 à 40 mm de diamètre, pour une lecture plus précise du réflexe controlatéral.

Les résultats obtenus en utilisant l'appareil selon l'invention sur un échantillon de 500 patients volontaires peuvent être résumés comme suit, exprimés en % d'amélioration par rapport à la pathologie de départ :
phénomènes douloureux : 80% de résultats positifs en 4 séances espacées de 48 heures.
blocages vertébraux : 60% à 75% de résultats positifs en 4 à 5 séances espacées de 48 heures, à condition que le blocage vertébral ne soit pas en liaison directe avec une hernie discale ou avec un processus mécanique quelconque.
allergies : 45% à 55% de résultats positifs en une dizaine de séances, pratiquées à raison de 3 par semaine.
troubles circulatoires et troubles métaboliques :
   30% à 40% d'amélioration des états pathologiques, toujours en thérapeutique adjuvante ; le nombre de séances variant entre 5 et 10 séances consécutives.
cures esthétiques : 45% d'amélioration dans les thérapies des acnés de l'adulte, 40% d'amélioration des douleurs observées dans les cas d'insuffisances veineuses des membres inférieurs ; le nombre de séances allant de 10 à 15, avec une fréquence de 2 par semaine.
cures relaxantes et anti-stress : 30% à 35% d'amélioration des états d'angoisse et des tableaux cliniques de dystonie neuro-végétative ; le nombre de séances étant compris entre 10 à 20, avec une fréquence de 3 par semaine.

Il est important de souligner ici la spécificité de la technique de chromo-réflexologie, où chaque patient réagit de façon parfaitement individualisé. Par exemple, pour un phénomène douloureux rencontré lors d'une sciatique, le vert pourra soulager un patient, tandis que c'est le rouge qui en améliorera un autre. La chromo-réflexologie substitue à une pratique codifiée (la chromothérapie) une pratique quantifiée en fonction d'un phénomène réflexe, pulsologie ou réflexe pupillaire par exemple.

## Revendications

1. Appareil de chromo-réflexologie, caractérisé en ce qu'il comprend une source (1) de lumière polychromatique, des moyens (6) pour disperser cette lumière et en réaliser un spectre (10), au moins un guide d'onde (11) reprenant à partir de la lumière dispersée un faisceau monochromatique, l'extrémité de sortie (13) du guide d'onde conduisant la lumière monochromatique à proximité du point de traitement, et des moyens pour balayer le spectre de façon continu avec l'extrémité d'entrée (12) du guide d'onde, pour sélectionner ainsi et à volonté la longueur d'onde de la lumière reprise dans le guide d'onde (11) et la conduire vers le point de traitement, qui correspond à une zone réflexe du corps.

2. Appareil de chromo-réflexologie selon la revendi- i-cation 1, caractérisé en ce qu'il comprend un disque tournant (4) percé de trous, placé sur le chemin lumineux, afin d'interrompre périodiquement celui-ci et de créer à la sortie un faisceau coloré pulsé.

3. Appareil de chromo-réflexologie selon la revendication 1 ou 2, caractérisé en ce que le balayage du spectre (10) se fait à l'aide de moyens mécaniques déplaçant l'extrémité d'entrée (12) du guide d'onde (11) dans le spectre (10).

4. Appareil de chromo-réflexologie selon la revendication 1 ou 2,caractérisé en ce que le guide d'onde (11) est une fibre optique.

5. Appareil de chromo-réflexologie selon la revendi- i-cation 4,caractérisé en ce que le guide d'onde est une fibre optique ayant un diamètre de 1 mm.

6. Appareil de chromo-réflexologie selon la revendication 1 ou 2, caractérisé en ce que les moyens de dispersion (6) consistent en un réseau.

7. Appareil de chromo-réflexologie selon la revendication 6, caractérisé en ce que le réseau comprend 600 lignes par mm.

8. Appareil de chromo-réflexologie selon l'une des revendications précédentes, caractérisé en ce qu'il comprend un dispositif optique complémentaire permettant de travailler loin de la cornée.

9. Appareil de chromo-réflexologie selon l'une des revendications précédentes, caractérisé en ce qu'il comprend une deuxième fibre optique reprenant la lumière dispersée au même point que la première fibre optique, l'appareil permettant alors l'éclairage simultané de deux zones différentes par une lumière de même longueur d'onde.
